(19) **Europäisches Patentamt / European Patent Office / Office européen des brevets**

(11) **EP 3 570 854 B1**

(12) **FASCICULE DE BREVET EUROPEEN**

(45) Date de publication et mention
de la délivrance du brevet:
**13.03.2024 Bulletin 2024/11**

(21) Numéro de dépôt: **18702769.3**

(22) Date de dépôt: **17.01.2018**

(51) Classification Internationale des Brevets (IPC):
**A61K 35/64** (2015.01)   **A23L 33/10** (2016.01)
**A23K 50/80** (2016.01)   **A61P 31/04** (2006.01)
**A61K 35/63** (2015.01)

(52) Classification Coopérative des Brevets (CPC):
**A61P 31/04; A23K 10/20; A23K 20/147;
A23K 20/163; A23K 40/10; A23K 50/80;
A23L 33/10; A61K 35/63**

(86) Numéro de dépôt international:
**PCT/FR2018/050119**

(87) Numéro de publication internationale:
**WO 2018/134524 (26.07.2018 Gazette 2018/30)**

(54) **UTILISATIONS THÉRAPEUTIQUES D'UNE POUDRE D'INSECTES**

THERAPEUTISCHE VERWENDUNGEN EINES INSEKTENPULVERS

THERAPEUTIC USES OF AN INSECT POWDER

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **18.01.2017 FR 1750390
30.06.2017 FR 1756099**

(43) Date de publication de la demande:
**27.11.2019 Bulletin 2019/48**

(73) Titulaire: **Ynsect
91058 Evry Cedex (FR)**

(72) Inventeurs:
• **MOTTE, Constant
59510 Hem (FR)**
• **ARMENJON, Benjamin
75013 Paris (FR)**
• **HUBERT, Antoine
94140 Alfortville (FR)**
• **BEREZINA, Nathalie
75005 Paris (FR)**

(74) Mandataire: **Santarelli
Tour Trinity
1 bis Esplanade de la Défense
92035 Paris La Défense Cedex (FR)**

(56) Documents cités:
**WO-A1-2016/108036    WO-A1-2016/108037**

• **DATABASE WPI Week 201449 Thomson
Scientific, London, GB; AN 2014-N93297
XP002774497, & CN 103 815 167 A (DALIAN
DETONG BIOTECHNOLOGY DEV CO LTD) 28 mai
2014 (2014-05-28)**
• **DATABASE WPI Week 201711 Thomson
Scientific, London, GB; AN 2016-71086U
XP002774495, & CN 106 071 395 A (YANG C) 9
novembre 2016 (2016-11-09)**
• **DATABASE WPI Week 201617 Thomson
Scientific, London, GB; AN 2015-837936
XP002774496, & CN 105 106 753 A (CHONGQING
FUJIANG BIOLOGICAL TECHNOLOGY) 2
décembre 2015 (2015-12-02)**

**Description**

La présente invention se rapporte à une poudre d'insectes et à son utilisation, notamment en aquaculture.

L'aquaculture est aujourd'hui l'un des secteurs les plus dynamiques de l'industrie alimentaire. L'aquaculture concerne notamment les productions de poissons (pisciculture), de coquillages (conchyliculture) ou encore de crustacés (astaciculture et pénéiculture).

La forte demande en poissons a eu pour conséquence d'augmenter significativement le prix des aliments destinés à l'élevage des poissons, coquillages et crustacés.

L'un des produits les plus utilisé dans l'aquaculture est la farine de poisson. C'est une farine très riche en protéines animales (riche en acides aminées type lysine et méthionine), facile à digérer. Une demande croissante accompagnée d'une offre limitée a eu pour conséquence d'un augmenter significativement son prix, engendrant un risque pour la croissance durable de l'aquaculture. Ainsi, il y existe une forte demande en sources alternatives de protéines de qualité élevée, et dans la mesure du possible, renouvelables, pour les aliments de l'aquaculture.

Les poudres d'insectes ou farine d'insectes proposent des sources protéiques naturelles de remplacement, ainsi que la possibilité d'être produite en masse avec une empreinte écologique minimale. En particulier, certains coléoptères tels que *Tenebrio molitor* présentent l'intérêt de pouvoir être adaptés à une production en masse intensive.

Par ailleurs, les élevages de poissons, de coquillages ou de crustacés, du fait du regroupement d'un nombre important d'individus dans les bassins d'élevage, sont très fréquemment assujettis à des épidémies d'origine bactérienne telle que par exemple *Vibrio parahaemolyticus* qui a décimé plus de 70% de la population de crevettes d'élevage dans certaines régions.

*Vibrio parahaemolyticus* est une bactérie qui infecte principalement les coquillages (notamment les huîtres et les moules), les crustacés (en particulier les crevettes) et les poissons. Cette bactérie est transmissible à l'homme, notamment lors de l'ingestion de coquillage, crustacé ou poisson infecté et cause des entérites et gastro-entérites.

Il existe donc un besoin en un moyen efficace de prévenir le risque d'infections et/ou de combattre efficacement les épidémies bactériennes.

A cet effet, les inventeurs ont mis en évidence que l'administration de poudre d'insectes permettait prévenir et de guérir des infections bactériennes.

Par « poudre d'insectes», on entend une composition, sous forme de particules, préparée uniquement à partir d'insectes et éventuellement d'eau.

Le taux d'humidité résiduel de la poudre d'insectes est compris entre 2 et 15%, de préférence entre 5 et 10%, plus préférentiellement, entre 4 et 8%. Ce taux d'humidité peut par exemple être déterminé selon la méthode issue du règlement CE 152/2009 du 27-01-2009 (103 °C / 4 h).

On notera que dans le cadre de la présente demande, et sauf stipulation contraire, les gammes de valeurs indiquées s'entendent bornes incluses.

Dans toute la demande, lorsqu'aucune date n'est précisée pour un règlement, une norme ou une directive, il s'agit du règlement, de la norme ou de la directive en vigueur à la date de dépôt.

Lorsque la poudre d'insectes est broyée à une taille de particules acceptable pour l'alimentation humaine ou animale, celle-ci peut être désignée sous l'appellation « farine d'insectes». Par « taille de particules acceptable pour l'alimentation humaine ou animale », on vise une taille de particules comprise entre 100 µm et 1,5 mm, préférentiellement comprise entre 300 µm et 1 mm, plus préférentiellement entre 500 et 800 µm.

Par « insectes », on désigne notamment les coléoptères, les diptères, les lépidoptères, les orthoptères, les hyménoptères, les dictyoptères regroupant notamment les blattoptères, y inclus isoptères, et les mantoptères, les phasmoptères, les hémiptères, les hétéroptères, les éphéméroptères et les mécoptères, de préférence, les coléoptères ; ou leurs mélanges.

Selon l'invention, la poudi d'insectes est un poudre de coléoptères, dans laquelle les coléoptères appartiennent aux familles des Tenebrionidae, Melolonthidae , Dermestidae, Coccinellidae, Cerambycidae, Carabidae, Buprestidae, Cetoniidae, Dryophthoridae, ou leurs mélanges.

Plus préférentiellement, il s'agit des coléoptères suivants : *Tenebrio molitor, Alphitobius diaperinus, Zophobas morio, Tenebrio obscurus, Tribolium castaneum et Rhynchophorus ferrugineus ;* ou leurs mélanges.

Avantageusement, la poudre d'insectes est obtenue à partir du stade larvaire des espèces d'insectes visées ci-avant.

La poudre d'insectes selon la description peut être administrée à l'animal en vue d'un traitement préventif et/ou curatif.

Cette poudre d'insectes permet notamment de lutter efficacement contre les infections bactériennes causées par des bactéries du genre *Vibrio species,* en particulier les infections bactériennes causées par *Vibrio parahaemolyticus.*

En effet, lorsque l'on nourrit des crevettes blanches infectées par *Vibrio parahaemolyticus* avec de la poudre d'insectes plutôt qu'avec de la farine de poisson, le taux de mortalité des crevettes blanches s'en trouvait limité. Cela résulte probablement d'un renforcement des défenses immunitaires des crevettes blanches, due à une stimulation leur système immunitaire et/ou à un meilleur développement de leur cellules hépatocytaires (voir Exemple 4 ci-après).

La présente invention vise donc une poudre de coléoptères pour une utilisation dans le traitement de la vibriose chez certains mollusques et crustacés spécifiques, à savoir les bivalves et les décapodes, les coléoptères appartenant aux familles des Tenebrionidae, Melolonthidae, Dermestidae, Coccinellidae, Cerambycidae, Carabidae, Buprestidae, Cetoniidae, Dryopthoridae, ou leurs mélanges.

Par « vibriose », on entend toutes infections bactériennes causées par des bactéries du genre *Vibrio spp*, appartenant à la famille des *Vibrionaceae*, bactéries vivant dans l'eau. Comme indiqué ci-avant, ces bactéries possèdent un pouvoir pathogène contre les hommes (pouvant créer des infections graves) et/ou contre les animaux, notamment les animaux aquatiques pouvant ainsi contaminer l'homme par le biais de son alimentation.

De préférence, ces bactéries sont *Vibrio aestuarianus* responsable de la maladie de l'huître, *Vibrio parahaemolyticus* responsable d'entérites et de gastro-entérites ou *Vibrio splendidus* responsable de la maladie infectieuse de l'huître creuse. De préférence, la poudre d'insectes est destinée à une utilisation dans le traitement de la vibriose causée par *Vibrio parahaemolyticus.*

Par « mollusques », on désigne un animal invertébré à corps mou, composé d'une tête, d'un pied et d'une masse viscérale. Parmi les sept classes de mollusques, on peut citer trois grands embranchements : les gastéropodes, les bivalves et les céphalopodes.

Les gastéropodes regroupent notamment les escargots, les limaces ou encore les patelles. Les bivalves regroupent notamment les moules, les huîtres ou encore les palourdes. Les céphalopodes regroupent notamment les poulpes, les calmars ou encore les seiches.

Les mollusques visés par l'invention peuvent être des animaux terrestres ou aquatiques, de préférence aquatiques. Par « aquatique », on entend des animaux d'eaux douces ou des animaux marins, de préférence marins.

Les mollusques visés par l'invention appartiennent aux groupes des bivalves comprenant notamment les *Palaeotaxodonta,* les *Cryptodonta,* les *Pteriomorphia,* les *Palaeoheterodonta,* les *Heterodonta* et les *Anomalodesmata.*

Préférentiellement, les mollusques sont choisis parmi les *Mytilida* (moules) et les *Ostreida* (huitres). Les *Ostreida* regroupent notamment les *Ostrea,* les *Crassostrea* (huîtres creuses), les *Ostreola* (huîtres plates) ou encore les *Saccostrea.*

En particulier, les espèces visées de mollusques sont : *Mytilus edulis, Mytilus galloprovincialis, Tapes rhomboides* (palourde rose), *Venerupis decussata* (palourde grise européenne), *Venerupis philippinarum* (palourde grise japonaise), *Venerupis aurea* (palourde dorée), *Corbicula fluminea* (palourde asiatique), *Ostrea edulis* (huître gravette), *Crassostrea angulata* (Huître portugaise), *Crassostrea gigas* (huître japonaise), *Ostreola conchaphila* (huître olympique), *Crassostrea virginica* (huître américaine ou huître de Virginie).

Par « crustacés », on désigne des animaux dont le corps est revêtu d'un exosquelette chitinoprotéique appelé exocuticule qui est souvent imprégné de carbonate de calcium. Ce groupe comporte des animaux de tailles variables appartenant aux classes inférieures telles que les *Branchiopoda,* les *Cephalocarida,* les *Malacostraca,* les *Maxillopoda,* les *Ostracoda,* les *Remipedia.*

Les espèces de crustacés visés par l'invention appartiennent à l'ordre des décapodes.

Par « décapodes », on désigne des crustacés possédant 5 paires de pattes. Les espèces de décapodes sont regroupées en deux sous-classes : les *Dendrobranchiata* et les *Pleocyemata.*

Parmi les *Dendrobranchiata,* on peut citer, de leur nom vernaculaire, les gambas appartenant à la super-famille des *Penaeoidea Rafinesque.*

Parmi les *Pleocyemata,* on peut citer, de leur nom vernaculaire, les galathées appartenant à l'infra-ordre des *Anomoure,* les crabes appartenant à la subsection des *Heterotremata* ou aux infra-ordres des *Brachyura* ou des *Paguroidea,* les crevettes appartenant à l'infra-ordre *Caridea,* les écrevisses et les homards appartenant à l'infraordre des *Astacidea* ou encore langoustes appartenant à l'infra-ordre des *Chelata.*

De préférence, les crustacés visés par l'invention sont des crevettes. Les crevettes peuvent être des espèces marines ou dulcicoles. De façon avantageuse, il s'agit d'espèces de crevettes adaptées à la péniculture ou à l'élevage d'eau douce.

Avantageusement, les espèces de crevettes visées par l'invention appartiennent à la famille des *Penaeidae* et notamment au genre *Penaeus.*

Préférentiellement, les espèces de crevettes visées par l'invention sont les suivantes : la crevette à pattes blanches (*Litopenaeus vannamei*), la crevette géante tigrée (*Penaeus monodon*), la crevette bleue (*Penaeus stylirostris*), la crevette charnue (*Penaeus chinensis*), la crevette impériale ou crevette japonaise (*Penaeus japonicus*), la crevette blanche des Indes (*Penaeus indicus*), la crevette banane (*Penaeus merguiensis*), la crevette akiami (*Metapenaeus spp*) et la crevette géante d'eau douce (*Macrobrachium rosenbergii*), encore préférentiellement *Litopenaeus vannamei.*

Les crevettes ont plusieurs stades de développement, trois stades larvaires, à savoir Nauplius, Zoés, Mysis, puis elles se métamorphosent en post-larves. La poudre d'insectes est particulièrement adaptée aux stades post-larvaire et/ou adulte.

L'invention concerne également une poudre d'insectes pour une utilisation dans le traitement de l'entérite de la crevette.

En particulier, la poudre d'insectes peut être utilisée pour une utilisation dans le traitement du syndrome de mortalité précoce de la crevette / syndrome de nécrose hépatopancréatique aiguë chez la crevette.

Le syndrome de mortalité précoce de la crevette / syndrome de nécrose hépatopancréatique aiguë (dit de « EMS/AHPNS ») est responsable d'un taux de mortalité chez la crevette infectée par *Vibrio parahaemolyticus* dépassant les 70%.

Lorsqu'elle est infectée, la crevette parait affaiblie et désorientée. Elle présente parfois des blessures noires sur sa cuticule. Certaines souches bactériennes très virulentes peuvent décimer jusqu'à 80% des crevettes d'élevages telles que les crevettes géantes tigrées (*Penaeus monodon*) et les crevettes à pattes blanches (*Penaeus vannamei*).

Avantageusement, la poudre d'insectes utilisée selon l'invention comporte au moins 67% en poids de protéines et au moins 0,1% en poids de chitine, les pourcentages étant donnés sur le poids total de poudre d'insectes.

Par « protéines », on vise la quantité de protéines brutes. La quantification des protéines brutes est bien connue de l'homme du métier. A titre d'exemple, on peut citer la méthode Dumas ou la méthode Kjeldhal. De préférence, la méthode Dumas, correspondant à la norme NF EN ISO 16634-1 (2008) est utilisée.

Des exemples d'une telle poudre sont décrits dans les Exemples 1 à 3 ci-après.

Préférentiellement, la poudre d'insectes comporte 68% en poids de protéines brutes, plus préférentiellement 70% en poids de protéines brutes, plus préférentiellement 71% en poids de protéines brutes, les pourcentages en poids étant donnés sur le poids total de poudre d'insectes.

Selon l'invention, par « chitine », on entend tout type de dérivé chitinique, c'est-à-dire de dérivé de polysaccharides comportant des unités N-acétyl-glucosamine et des unités D-glucosamines, en particulier les copolymères chitine-polypeptides (parfois désignés sous l'appellation « composite chitine-polypeptides »). Ces copolymères peuvent également être associés à des pigments, souvent de type mélanine.

La chitine serait le deuxième polymère le plus synthétisé dans le monde vivant après la cellulose. En effet, la chitine est synthétisée par de nombreuses espèces du monde vivant : elle constitue en partie l'exosquelette des crustacés et des insectes et la paroi latérale qui entoure et protège les champignons. Plus particulièrement, chez les insectes, la chitine constitue ainsi 3 à 60% de leur exosquelette.

La détermination du taux de chitine est effectuée par extraction de celle-ci. Une telle méthode peut être la méthode AOAC 991.43.

Selon un premier mode de réalisation, la poudre d'insectes utilisée selon l'invention comporte au moins 67% en poids de protéines et au moins 5% en poids de chitine, les pourcentages étant donnés sur le poids total de poudre d'insectes.

Préférentiellement, cette poudre d'insecte comporte entre 5 et 16% en poids de chitine, plus préférentiellement entre 8 et 14% de chitine, les pourcentages en poids étant donnés sur le poids total de poudre d'insectes.

Avantageusement, cette poudre d'insectes présente une teneur en cendres inférieure ou égale à 4% en poids sur le poids total de poudre d'insectes, et encore plus avantageusement inférieure ou égale à 3,5%.

Les cendres constituent le résidu résultant de la combustion de la composition selon l'invention.

La méthode de détermination de la teneur en cendres est bien connue de l'homme du métier. De préférence, les cendres ont été déterminées selon la méthode relevant du règlement CE 152/2009 du 27-01-2009.

La teneur en matière grasse de cette poudre d'insectes est de préférence comprise entre 5 et 20% en poids sur le poids total de poudre d'insectes, plus préférentiellement entre 9 et 17%.

Les méthodes de détermination de la teneur en matière grasse sont bien connues de l'homme du métier. A titre d'exemple et de manière préférée, la détermination de cette teneur sera effectuée en suivant la méthode du règlement CE 152/2009.

Avantageusement, les protéines de cette poudre d'insectes présentent une digestibilité supérieure ou égale à 85% en poids sur le poids total de protéines brutes.

La digestibilité est une digestibilité pepsique mesurée par la méthode décrite dans la directive 72/199/CE.

De préférence, la digestibilité est supérieure ou égale à 86%, plus préférentiellement, supérieure ou égale à 88%.

Avantageusement, cette poudre d'insectes utilisée selon l'invention comporte entre 35 et 65% en poids de protéines solubles par rapport au poids total de protéines, et au moins 50% des protéines solubles ont une taille inférieure ou égale à 12400g/mol.

Par « poids total de protéines », on entend le poids de protéines brutes présentes dans la poudre d'insectes selon l'invention.

Par « protéines solubles », on entend, parmi les protéines brutes, celles qui sont solubles dans une solution aqueuse dont le pH est compris entre 6 et 8, avantageusement entre 7,2 et 7,6.

De préférence, la solution aqueuse est une solution tampon dont le pH est compris entre 6 et 8, avantageusement entre 7,2 et 7,6. Préférentiellement, la solution tampon est une solution tampon phosphate NaCl, dont le pH est égal 7,4 +/- 0,2.

Avantageusement, cette poudre d'insectes comporte entre 38 et 60% en poids, de préférence entre 43 et 55% en poids de protéines solubles par rapport au poids total de protéines.

De préférence, au moins 60%, préférentiellement au moins 70% des protéines solubles ont une taille inférieure ou égale à 12400g/mol.

Plus particulièrement, les protéines solubles ont une taille comprise entre 6500 et 12400g/mol.

Avantageusement, moins de 10%, de préférence moins de 8%, plus préférentiellement moins de 6% de protéines solubles ont une taille supérieure ou égale à 29000g/mol.

Cette poudre d'insectes peut être préparée par un procédé comportant les étapes suivantes :

    i) abattage des insectes,
    ii) pressage des insectes pour obtenir un gâteau de presse, et
    iii) broyage du gâteau de presse.

L'abattage des insectes peut être effectué par ébouillantage ou blanchiment, comme cela est plus amplement décrit ci-après l'Exemple 1 ci-après.

De même, les étapes de pressage et de broyage sont plus amplement décrites dans cet Exemple.

Enfin, le procédé de préparation peut comporter en outre une étape de séchage du gâteau de presse.

L'étape de séchage est avantageusement réalisée après l'étape de pressage et avant l'étape de broyage et est également décrite plus amplement dans l'Exemple 1 ci-après.

Selon un second mode de réalisation, la poudre d'insectes utilisée selon l'invention comporte au moins 71% en poids de protéines et comporte entre 0,1 et 2% en poids de chitine, les pourcentages étant donnés sur le poids total de poudre d'insectes.

De préférence, cette poudre d'insectes a une teneur en protéines supérieure ou égale à 72% en poids, plus préférentiellement supérieure ou égale à 74% en poids, encore plus préférentiellement supérieure ou égale à 75% en poids, sur le poids sec total de poudre.

Plus particulièrement, cette poudre a une teneur en chitine comprise entre 0,5 et 3% en poids, plus préférentiellement comprise entre 0,8 et 2% en poids, encore plus préférentiellement comprise entre 0,8 et 1,7% en poids sur le poids sec total de poudre.

De préférence, cette poudre comprend entre 5 et 20% en poids, de préférence entre 7 et 17% en poids de lipides, sur le poids sec total de poudre.

Plus particulièrement, cette poudre comprend entre 1 et 10% en poids, de préférence entre 2 et 6% en poids de cendres, sur le poids sec total de poudre.

Avantageusement, les protéines de cette poudre d'insectes présentent une digestibilité supérieure ou égale à 85% en poids sur le poids total de protéines brutes.

De préférence, la digestibilité est supérieure ou égale à 88%, plus préférentiellement, supérieure ou égale à 92%.

Cette poudre d'insectes peut être préparée par un procédé comportant les étapes suivantes :

- abattage des insectes,
- la séparation des cuticules de la partie molle des insectes,
- la séparation de la partie molle des insectes en une fraction solide, une fraction huileuse et une fraction aqueuse,
- le séchage de la fraction solide pour obtenir une fraction solide sèche;
- le broyage de la fraction solide sèche pour obtenir une poudre d'insectes.

L'abattage des insectes peut être effectué par ébouillantage ou blanchiment, comme cela est plus amplement décrit ci-après l'Exemple 1 ci-après.

La cuticule est la couche externe (ou exosquelette) sécrétée par l'épiderme des insectes. Elle est en général formée de trois couches : l'épicuticule, l'exocuticule et l'endocuticule.

Par « partie molle », on vise la chair (comportant notamment les muscles et les viscères) et le jus (comportant notamment les liquides biologiques, l'eau et l'hémolymphe) des insectes. En particulier, la partie molle ne consiste pas en le jus des insectes.

La séparation des cuticules de la partie molle des insectes peut être effectuée à l'aide d'un filtre presse ou d'un séparateur à bande.

Par séparateur à bande, on vise un dispositif qui comporte une bande de serrage (ou bande presseuse) et un tambour perforé.

Dans la présente demande, la poudre d'insectes est obtenue à partir d'une espèce d'insectes appartenant à l'ordre des coléoptères, de préférence à partir de l'espèce *Tenebrio molitor,* et ce quel que soit le mode de réalisation de l'invention. La poudre de coléoptères est alors une poudre de *Tenebrio molitor.*

L'invention vise donc une poudre de coléoptères, et notamment une poudre de *Tenebrio molitor,* pour une utilisation :

- dans le traitement de la vibriose chez les bivalves et les décapodes,
- dans le traitement de l'entérite de la crevette, et/ou
- dans le traitement du syndrome de mortalité précoce de la crevette / syndrome de nécrose hépatopancréatique aigüe chez la crevette.

Il est également décrit en outre un régime alimentaire pour mollusques et/ou crustacés, comportant au moins 5 % en poids de poudre d'insectes non couvert par les revendications.

Par « régime alimentaire », on entend un ensemble d'ingrédients (ou constituants) alimentaires selon des proportions données, destinés à être consommés par les mollusques et/ou crustacés.

La poudre d'insectes peut être utilisée comme alternative à la farine de poisson généralement administrée dans le régime alimentaire des mollusques et des crustacés. Elle peut remplacer partiellement ou en totalité la farine de poisson. Préférentiellement, la poudre d'insectes remplace la farine de poisson à 50 % ou plus, en poids de farine de poisson, encore préférentiellement à 75 % ou plus, en poids de farine de poisson.

La substitution de la farine de poisson par de la poudre d'insectes permet avantageusement d'améliorer le système immunitaire des mollusques et/ou des crustacés, notamment du fait de la grande digestibilité des protéines et des lipides de la poudre d'insectes. En outre, cela augmente la densité, ainsi que la longueur des villosités intestinales, en particulier chez la crevette blanche tout en augmentant la taille des cellules hépatocytaires. Aussi, cette substitution de farine de poisson par de la poudre d'insectes présente une alternative riche en protéines et en lipides, peu onéreuse et qui permet de prévenir et/ou de guérir certaines maladies telles que la vibriose de la crevette.

Le régime alimentaire administré aux mollusques ou aux crustacés, comporte avantageusement au moins 10 % en poids de poudre d'insectes, préférentiellement au moins 15% en poids de poudre d'insectes, encore préférentiellement au moins 25% en poids de poudre d'insectes.

Selon un mode avantageux de réalisation, le régime alimentaire administré aux mollusques ou aux crustacés, comportant 20 % en poids de poudre d'insectes.

Préférentiellement, la poudre d'insectes remplace en totalité la farine de poisson généralement administrée aux mollusques et aux crustacés. Aussi, le régime alimentaire administré aux mollusques ou aux crustacés, comporte avantageusement 25 % en poids de poudre d'insectes.

Ce régime est particulièrement adapté aux crevettes, les autres constituants du régime, ainsi que leurs proportions, étant définis le manuel (« *Nutrient requirements of fish and shrimp* ») publié par le National Research Council (NRC) en 2011.

Avantageusement, le régime alimentaire comprend une poudre d'insectes comportant au moins 71% en poids de protéines et comprenant entre 0,1 et 2% en poids de chitine, les pourcentages étant donnés sur le poids total de poudre d'insectes.

De préférence, la poudre d'insectes est une poudre de coléoptères, plus préférentiellement de *Tenebrio molitor.*

Il est également décrit, l'utilisation d'une poudre d'insectes comportant au moins 67% en poids de protéines et comprenant au moins 0,1% en poids de chitine, les pourcentages étant donnés sur le poids total de poudre d'insectes, comme complément alimentaire dans la nutrition humaine ou animale, ce mode de réalisation étant non couvert par les revendications.

La poudre d'insectes utilisée comme complément alimentaire dans la nutrition humaine ou animale peut comporter au moins 67% en poids de protéines et au moins 5% en poids de chitine, les pourcentages étant donnés sur le poids total de poudre d'insectes. Il s'agit alors de la poudre d'insectes du premier mode de réalisation décrit ci-avant, y inclus toutes les caractéristiques avantageuses, particulières et préférées et son procédé d'obtention.

Alternativement, la poudre d'insectes utilisée comme complément alimentaire dans la nutrition humaine ou animale peut comporter au moins 71% en poids de protéines et comprenant entre 0,1 et 2% en poids de chitine, les pourcentages étant donnés sur le poids total de poudre d'insectes. Il s'agit alors de la poudre d'insectes du second mode de réalisation décrit ci-avant, y inclus toutes les caractéristiques avantageuses, particulières et préférées et son procédé d'obtention.

Avantageusement, la poudre d'insectes est une poudre de coléoptères, de préférence de *Tenebrio molitor.*

Il est également décrit l'utilisation d'une poudre d'insectes, notamment telles que décrites ci-avant, comme complément alimentaire dans la nutrition des mollusques et/ou des crustacés.

Il est également décrit un complément alimentaire comportant une poudre d'insectes telle que décrite ci-avant.

Avantageusement, la poudre d'insectes est utilisée comme complément alimentaire dans la nutrition des crevettes, en particulier des crevettes d'élevage.

D'autres caractéristiques et avantages de l'invention, apparaîtront dans les exemples qui suivent, donnés à titre illustratif, avec référence à :

- la Figure 1 est un diagramme illustrant le poids final des crevettes blanches nourries selon différents régimes alimentaires, comportant ou non de la poudre d'insectes à la place de la farine de poisson ;

- la Figure 2 est un diagramme illustrant le gain de poids des crevettes blanches nourries selon différents régimes alimentaires, comportant ou non de la poudre d'insectes à la place de la farine de poisson ;
- la Figure 3 est un diagramme illustrant l'activité de la phénol oxidase en fonction de la réduction bactérienne du pathogène, chez les crevettes blanches infectées par *Vibrio parahaemolyticus* et nourries selon différents régimes alimentaires, comportant ou non de la poudre d'insectes à la place de la farine de poisson ; et
- la Figure 4 est un diagramme illustrant le taux de survie des crevettes infectées par *Vibrio parahaemolyticus* en fonction du régime alimentaire apportés, comportant ou non de la poudre d'insectes à la place de la farine de poisson.

## EXEMPLE 1 : Procédé de préparation d'une poudre d'insectes

La composition selon l'invention est préparée à partir de larves de *Tenebrio molitor.* A réception des larves, ces dernières peuvent être stockées à 4°C pendant 0 à 15 jours dans leurs bacs d'élevages avant l'abattage sans dégradation majeure. Le poids des larves par rapport à l'âge des larves utilisées est variable et par conséquent leur composition peut varier, comme cela est illustré dans le Tableau 1 ci-après :

**Tableau 1** : Composition biochimique des larves de *Tenebrio molitor* selon leur poids.

| Biomasse (Insectes) | mg | 23 | 35 | 58 | 80 | 108 | 154 |
|---|---|---|---|---|---|---|---|
| Matière sèche | %* | 34 | 34 | 34,2 | 37,9 | 39,6 | 39,5 |
| Cendres | %* | 1,59 | 1,52 | 1,6 | 1,75 | 1,67 | 1,43 |
| Protéines brutes | %* | 22,6 | 22,2 | 22 | 23,2 | 23,1 | 23,2 |
| Lipides | %* | 6,62 | 6,88 | 7,98 | 10,3 | 10,9 | 11,7 |
| * Les % sont exprimés en poids sec par rapport au poids humide de larves. | | | | | | | |

### • Etape 1 : Blanchiment des insectes

Les larves vivantes (+ 4°C à + 25°C) sont convoyées en couche d'épaisseur comprise entre 2 et 10 cm, sur un tapis à bande perforé (1mm) jusqu'à une chambre de blanchiment. Les insectes sont ainsi blanchis à la vapeur (buses ou lit de vapeur) à 98°C ou bien à l'eau à 100°C (buses d'aspersion) ou en mode mixte (eau + vapeur). Le temps de séjour dans la chambre de blanchiment est compris entre 1 à 15 minutes, idéalement 5 min.
La température des larves en sortie de blanchiment est comprise entre 75°C et 98°C.

### • Etape 2 : Pressage

Les larves, une fois blanchies, sont convoyées jusqu'à la trémie d'alimentation d'une presse mono-vis continue. Les larves lors du passage en presse sont maintenues à une température supérieure à 70°C pour augmenter les rendements de déshuilage. Le principe de déshuilage est de mettre en pression la matière à l'intérieur d'une cage cylindrique au moyen d'un arrangement de vis et de bagues disposées sur l'axe central. La cage est tapissée à l'intérieur de barreaux répartis en section et maintenus écartés par des espaces d'épaisseurs différentes suivant la zone de travail. Les interstices ainsi ménagés permettent l'écoulement d'une fraction huile et limitent le passage de la matière dite « sèche », la fraction protéique, que l'on appellera « gâteau de presse », participant de la sorte à la mise en pression.
Les rendements de pressage obtenus sont compris entre 48 et 55 %.

$$R_{gâteau} = (masse_{gâteau} / masse_{jus} + masse_{gâteau})$$

Le gâteau de presse obtenu contient 35 à 40 % de matières sèches, 67 à 75 % de protéines et 13 à 17 % de matières grasses, les pourcentages en poids étant donnés sur le poids sec de gâteau de presse.

### • Etape 3 : Séchage

Le gâteau de presse est ensuite disposé sur plateau en couche fine (2 cm environ) et, est séché en air ventilé/brassé à 90°C pendant 5 heures afin d'obtenir un gâteau de presse ayant une teneur en matière sèche supérieure à 92%.
Cette étape permet de se prémunir de toute contamination ayant eu lieu depuis l'abattage.

L'Aw (activité en eau) en sortie séchage est de 0,35. Les résultats microbiologiques montrent une absence de Salmonella spp (méthode : IRIS Salmonella BKR 23/07-10/11) et des valeurs en Entérobactéries inférieures à 10 UFC/g (méthode : NF ISO 2128-2, décembre 2004, 30°C et 37°C).

**• Etape 4 : Broyage**

Le gâteau de presse séché, comportant majoritairement des protéines, est enfin broyé à l'aide d'un broyeur à marteau continu (6 mobiles réversibles - épaisseur 8 mm). Le broyeur est alimenté par une trémie avec trappe de réglage de débit (180kg/h). La grille perforée utilisée pour contrôler la granulométrie en sortie est de 0,8 mm. La vitesse de rotation du moteur est de 3000tr/min (motorisation électrique, puissance absorbée 4kW (5,5 CV)).

**EXEMPLE 2 : Caractérisation de la poudre d'insectes obtenue dans l'Exemple 1**

La poudre d'insectes préparée à l'Exemple 1 a été caractérisée.

**1. Analyses**

1.1 Détermination du taux d'humidité

Le taux d'humidité est déterminé selon la méthode issue du règlement CE 152/2009 du 27-01-2009 (103 °C / 4 h).

1.2 Détermination de la quantité de protéines brutes

Les protéines brutes sont déterminées selon la méthode, dite de Dumas, et correspondant à la norme NF EN ISO 16634-1 (2008).

1.3 Détermination de la quantité de chitine

Les fibres alimentaires de la farine des insectes sont essentiellement composées de chitine, cette dernière a donc été dosée suivant la méthode AOAC 991.43. Les valeurs ainsi obtenues sont par conséquent légèrement surestimées.

1.4 Détermination de la quantité de matière grasse

La matière grasse a été déterminée suivant la méthode du règlement CE 152/2009.

1.5 Détermination de la quantité de cendres

Les cendres brutes ont été déterminées selon la méthode relevant du règlement CE 152/2009 du 27-01-2009.

1.6 Détermination de la quantité de phosphore

Le phosphore est dosé par ICP (« induced coupled plasma ») avec étalonnage interne.

1.7 Détermination de l'énergie

La valeur énergétique est obtenue avec les coefficients du règlement UE 1169/201.

1.8 Détermination des quantités en acides aminés et en acides gras

Cette détermination a été effectuée par chromatographie en phase gazeuse après hydrolyse et dérivatisation des acides aminés et acides gras respectivement.

1.9 Détermination de la digestibilité pepsique

La digestibilité pepsique est mesurée par la méthode décrite dans la directive 72/199/CE.

## 2. Résultats

La poudre d'insectes est détaillée dans le Tableau 2 ci-après.

**Tableau 2 :** Composition de la poudre d'insectes

| Macronutriment | Unité | Composition | Acides gras | Unité | Composition |
|---|---|---|---|---|---|
| Humidité | %* | 5,32 | C12:0 | %* | 0,03 |
| Protéine | %* | 67,09 | C14:0 | %* | 0,22 |
| Chitine | %* | 8,0 | C15:0 | %* | 0,01 |
| Matière grasse | %* | 13,6 | C16:0 | %* | 1,33 |
| Cendre | %* | 3,21 | C16:1 | %* | 0,05 |
| Phosphore total | %* | 0,75 | C16:1n-7 | %* | 0,16 |
| Énergie | MJ/kg | 23,74 | C17:0 | %* | 0,02 |
| | | | C17:1 | %* | 0,01 |
| **Acides aminés** | **Unité** | **Composition** | C18:0 | %* | 0,35 |
| Arginine | %* | 2,56 | C18:1n-9 | %* | 3,03 |
| Histidine | %* | 1,39 | C18:1n-7 | %* | 0,04 |
| Isoleucine | %* | 2,11 | C18:2n-6 | %* | 2,96 |
| Leucine | %* | 3,99 | C18:2tn-6 | %* | 0,02 |
| Lysine | %* | 3,32 | C18:3n-3 | %* | 0,14 |
| Thréonine | %* | 1,87 | C20:0 | %* | 0,02 |
| Valine | %* | 2,91 | C20:1n-9 | %* | 0,01 |
| Méthionine | %* | 1,43 | C20:2n-6 | %* | 0,01 |
| Cystéine | %* | 0,63 | C22:0 | %* | 0,01 |
| Phénylalanine | %* | 1,98 | | | |
| Tyrosine | %* | 2,68 | | | |
| Taurine | %* | 0,42 | | | |
| Acide aspartique + asparagine | %* | 4,51 | | | |
| Acide glutamique + glutamine | %* | 6,36 | * Les pourcentages en poids sont exprimés sur le poids total de composition de poudre. | | |
| Alanine | %* | 3,83 | | | |
| Glycine | %* | 2,54 | | | |
| Proline | %* | 3,18 | | | |
| Serine | %* | 2,94 | | | |

Par ailleurs, une digestibilité pepsique de 90+/-2% est obtenue.

## EXEMPLE 3 : Procédé alternatif de préparation d'une poudre d'insectes

200 g de larves de *T. molitor* sont introduits dans un bécher, placé dans un bain-marie à 100°C et contenant 200 mL d'eau préalablement portée à ébullition. Après 5 minutes, le bécher est retiré du bain-marie, les larves sont essorées, puis mixées avec un volume d'eau de 200 mL. Le liquide ainsi obtenu est passé dans une presse de type bi-vis. Le gâteau de presse ainsi obtenu est séché 24 heures dans une étuve à 70°C, puis broyé à 250 μm. On obtient ainsi une poudre d'insectes.

EXEMPLE 4 : Effets de la poudre d'insectes obtenue à l'Exemple 1 sur les crevettes blanches, notamment sur leur résistance à la bactérie pathogène *vibrio parahaemolyticus*

Trois expériences ont été menées afin d'évaluer l'appétence des poudres d'insectes chez la crevette (expérience 1), la digestibilité des protéines, des lipides et de l'énergie des poudres d'insectes chez la crevette (expérience 2) et l'efficacité de la poudre d'insectes sur la croissance et l'immunité de la crevette blanche (expérience 3).

**1. Matériel et méthode des expériences menées**

La farine de poisson utilisée pour l'ensemble de ces expériences est une farine de poisson provenant de Thaïlande (« Fish meal ») et comportant un taux de protéines brutes de 56 %, exprimé sur le poids total de ladite farine de poisson.
De la même manière, la poudre d'insectes utilisée est identique pour l'ensemble de ces expériences et correspond à la poudre d'insecte obtenue à l'Exemple 1.

**Statistiques**

Ces expériences ont été conçues de manière complètement aléatoire (CMCA). Toutes les données ont été analysées par ANOVA (analyse de variance). Le test à gamme multiple de Duncan a été utilisé pour déterminer les différences entre les moyennes des différents régimes alimentaires. La notation alphabétique a été utilisée pour marquer les différences au niveau significatif d'un alpha 0,05. Toutes les recherches des expériences 2 et 3 ont été menées au Laboratoire de nutrition et aliment pour animaux aquatiques, Département de l'aquaculture, Faculté des Pêches, Université Kasetsart, à Bangkok en Thaïlande.

***Expérience 1: Test d'appétence des poudres d'insectes sur la crevette blanche par rapport à un régime alimentaire usuel constitué de 15 % de farine de poisson.***

a. Les différents régimes alimentaires de l'expérience.

La capacité d'attraction des poudres d'insectes sur la crevette blanche a été évaluée de manière complètement aléatoire à l'aide des 5 régimes suivants, chacun répété quatre fois :

- Régime 1 (R1) : Contrôle 30 % farine de poisson (ci-après nommée FM) ;
- Régime 2 (R2) : Contrôle négatif 15 % de FM ; 0 % de poudre d'insectes (ci-après nommée INSM) ;
- Régime 3 (R3) : Contrôle négatif 10 % de FM ; 5% de INSM ;
- Régime 4 (R4) : Contrôle négatif 5% de FM ; 10% de INSM ;
- Régime 5 (R5) : Contrôle négatif 0 % de FM ; 15% de INSM ;

Quatre réplicas de chaque régime ont été utilisés pour évaluer l'appétence.

b. Formulations et production des régimes alimentaires

Les régimes ont été formulés avec des ingrédients utiles pour répondre aux besoins nutritionnels connus des crevettes blanches, notamment décrit dans le manuel de référence du National Research Council (NCR) publié en 2011.
La poudre d'insectes utilisée dans les régimes alimentaires expérimentaux est celle obtenue selon l'Exemple 1.
La capacité d'attraction des poudres d'insectes sur la crevette blanche a été évaluée dans cinq régimes alimentaires détaillés ci-dessus et dont la composition est présentée dans le Tableau 3 ci-après.
Les compositions approximatives des aliments à l'essai telles que l'humidité, les protéines, les lipides, les fibres, les cendres ont été analysées conformément à la description de l'AOAC (2000), qui est la méthode internationale de référence.

**Tableau 3** : Compositions des régimes alimentaires des crevettes blanches contenant de la poudre d'insectes à la place de la farine de poisson

| Ingrédient de la formule | R1 INSM 0 % - FM 30 % | R2 INSM 0 % - FM 15 % | R3 INSM 5 % - FM 10% | R4 INSM 10 % - FM 5 % | R5 INSM 15 % - FM 0 % |
|---|---|---|---|---|---|
| Farine de poisson | 30,0 | 15,0 | 10,0 | 5,0 | 0,0 |
| Soja | 15,0 | 15,0 | 15,0 | 15,0 | 15,0 |
| Gluten de blé | 8,0 | 8,0 | 8,0 | 8,0 | 8,0 |
| Poudre de foie de calmar | 8,0 | 8,0 | 8,0 | 8,0 | 8,0 |
| Farine de blé | 24,0 | 24,0 | 24,0 | 24,0 | 24,0 |
| Brisures de riz | 2,2 | 1,2 | 1,2 | 1,2 | 1,2 |
| Concentré de protéine de soja | 0,0 | 15,0 | 15,0 | 15,0 | 15,0 |
| Poudre d'insectes | 0,0 | 0,0 | 5,0 | 10,0 | 15,0 |
| Gluten de maïs | 5,0 | 5,0 | 5,0 | 5,0 | 5,0 |
| Huile de thon | 0,5 | 0,5 | 0,5 | 0,5 | 0,5 |
| Huile de soja | 2,0 | 2,0 | 2,0 | 2,0 | 2,0 |
| Lécithine de soja | 2,0 | 2,0 | 2,0 | 2,0 | 2,0 |
| Phosphate monocalcique | 0,0 | 1,0 | 1,0 | 1,0 | 1,0 |
| Liant | 1,7 | 1,7 | 1,7 | 1,7 | 1,7 |
| Prémix de vitamines | 1,6 | 1,6 | 1,6 | 1,6 | 1,6 |
| **Somme** | 100,0 | 100,0 | 100,0 | 100,0 | 100,0 |
| Composition chimique par (AOAC, 2000) | | | | | |
| Humidité (%) | 7,38 | 7,14 | 7,09 | 7,25 | 7,18 |
| Protéines (%) | 37,38 | 36,88 | 37,06 | 37,84 | 38,81 |
| Lipides (%) | 7,18 | 6,50 | 6,97 | 7,23 | 7,60 |
| Fibre (%) | 2,69 | 2,95 | 3,09 | 3,33 | 3,68 |
| Cendre (%) | 10,39 | 8,02 | 7,70 | 6,58 | 5,96 |
| Énergie (Kcal/Kg) | 4560,14 | 4467,36 | 4477,59 | 4487,82 | 4498,05 |

c. Protocole de nourrissage des crevettes blanches

Les crevettes blanches ont été nourries par des granulés contenant 36 % de protéines brutes et 7 % de lipides bruts, à une quantité d'environ 5 % du poids corporel moyen des crevettes/jour (soit 1,5-2 % du poids corporel / repas) en déposant les aliments sur un plateau et en appliquant 3 repas par jour.

Durant 10 jours et uniquement lors du repas de 13h00, le temps nécessaire aux crevettes pour ingérer initialement la nourriture, le nombre de crevettes atteignant la nourriture dans les 15 minutes qui suivent le dépôt des aliments sur le plateau et la consommation des aliments pour un repas ont été enregistrés.

Quatre réplicas de chaque régime ont été réalisés pour évaluer la capacité d'attraction.

La nourriture non consommée a été recueillie une heure après son dépôt à partir du plateau et séchée par sublimation à -40°C (« freeze-dried »), puis pesée pour déterminer la consommation alimentaire totale.

Sur une étude de 10 jours, la ration alimentaire de chaque plateau dans chaque régime a été calculée pour déterminer l'appétence, en se focalisant sur le temps nécessaire pour ingérer l'aliment, la quantité d'aliments consommée et le nombre de crevettes consommant les aliments.

### Expérience 2 : Étude de la digestibilité des protéines, des lipides et de l'énergie des poudres d'insectes chez la crevette blanche

La digestibilité *in vivo* des protéines, des lipides et de l'énergie de la poudre d'insectes chez la crevette blanche ont été déterminées par une méthode indirecte. Trois cents crevettes avec un poids moyen de 5 à 8 g ont été réparties de façon aléatoire dans chacun des vingt aquariums. Dix répétitions avec dix crevettes, chacune nourrie avec un régime de référence alimentaire composé de 25 % de farine de poisson et de 5% de farine de calmar, de 63,7 % de farine de blé, de gluten et d'isolat de soja et lécithine de soja, de 3 % d'un mélange d'huile de thon et de soja, de 3,3 % de prémix de vitamines et de 1 % d'oxyde chromique comme marqueur de digestibilité.

Un autre régime alimentaire a été fourni aux 200 autres crevettes (20 répétitions de 10 crevettes), composé de 30 % de poudre d'insectes plus 1 % d'oxyde chromique comme marqueur, de 63,7 % de farine de blé, de gluten et d'isolat de soja et lécithine de soja, de 3 % d'un mélange d'huile de thon et de soja, de 3,3 % de prémix de vitamines et de 1 % d'oxyde chromique comme marqueur de digestibilité.

La période d'acclimatation était d'une semaine. Après cette période, les crevettes ont été nourries avec les régimes alimentaires affectés en excès (4 %) trois fois par jour. Deux heures après le repas, les aquariums ont été nettoyés et une demi-heure avant le repas suivant, les matières fécales ont été collectées pendant 1 à 2 semaines.

Les échantillons provenant de chaque régime alimentaire ont été rassemblés à la fin de l'expérience de manière à avoir assez de matière pour l'analyse. Ils ont été séchés dans un four à air chaud à 65 °C. Les échantillons des aliments et des matières fécales ont été analysés pour les lipides et les protéines (AOAC, 2000). L'oxyde chromique a été déterminé (Scott, 1978). Le coefficient de digestibilité apparente (CDA) a été calculé tel que décrit par Cho et al. (1985).

- Coefficient de digestibilité apparente (CDA %) = 100-(Indicateur dans le régime alimentaire en %) / Indicateur dans les matières fécales en %
- Coefficient de digestibilité apparente des nutriments (CDA Nu %) = 100-(Indicateur dans le régime alimentaire en % X nutriments dans les matières fécales en %) / Indicateur dans les matières fécales en % X nutriment dans le régime alimentaire en %)

### Expérience 3 : Efficacité de la poudre d'insectes sur la performance de croissance et l'immunité de la crevette blanche (Litopenaeus vanammei)

a. Conception des essais

L'expérience a été conçue de manière complètement aléatoire (CMCA) de 5 régimes avec 4 réplicas.

Les différents régimes alimentaires sont indiqués dans le Tableau 4 ci-dessous.

**Tableau 4** : Les différents régimes alimentaires

| Régime alimentaire | Description | Niveau de farine de poisson dans le régime alimentaire | Poudre d'insectes remplaçant les protéines de la farine de poisson |
|---|---|---|---|
| T1 | INSM remplace 0 % de FM | 25 % | 0% |
| T2 | INSM remplace 25 % de FM | 18,75 % | 25 % (5,15 % INSM) |
| T3 | INSM remplace 50 % de FM | 12,5 % | 50 % (10,30 % INSM) |
| T4 | INSM remplace 75 % de FM | 6,25% | 75 % (15,40 % INSM) |
| T5 | INSM remplace 100 % de FM | 0 % | 100 % (20,50 % INSM) |

L'expérience a été réalisée dans 20 aquariums en verre ayant chacun des capacités de 100 litres dont 70 litres d'eau saline à 15 ppt (partie par trillion). Des crevettes blanches juvéniles (env. 3 à 4 g) ont été stockées à une densité de 40 crevettes/m$^2$ (10 par aquarium). La poudre d'insectes a été mélangée dans l'alimentation tandis que l'huile de poisson a été réduite graduellement. Les aliments ainsi préparés ont été appliqués aux crevettes 3 fois par jour à une quantité comprise entre 3 et 5 % du poids corporel moyen des crevettes pendant 8 semaines. Tous les 2 à 3 jours, les déchets de nourriture étaient siphonnés et l'eau était renouvelée à 15 - 20 %.

Les ingrédients des différents régimes alimentaires ont été broyés à 150-250 microns, mélangés ensemble, puis 25 % d'eau a été ajoutée avant le passage par le hachoir Hobart. Les granulés ainsi obtenus ont été séchés dans un four à air chaud.

Les compositions approximatives des régimes alimentaires testés telles que l'humidité, les protéines, les lipides, les fibres, les cendres et l'énergie ont été analysées conformément à la description de l'AOAC (2000). Cette composition chimique ainsi que celle en ingrédients sont résumés dans le Tableau 5 ci-dessous.

**Tableau 5** : Composition des régimes alimentaires expérimentaux b. Paramètres

| Matière | T1 INSM remplace 0 % de FM | T2 INSM remplace 25 % de FM | T3 INSM remplace 50 % de FM | T4 INSM remplace 75 % de FM | T5 INSM remplace 100 % de FM |
|---|---|---|---|---|---|
| Farine de poisson | 25 | 18,75 | 12,5 | 6,25 | 0 |
| Soja | 25 | 25 | 25 | 25 | 25 |
| Gluten de blé | 10 | 10 | 10 | 10 | 10 |
| Poudre de foie | 5 | 5 | 5 | 5 | 5 |
| Farine de blé | 26,7 | 26,6 | 26,65 | 26,15 | 25,55 |
| Poudre d'insectes | 0 | 5,15 | 10,3 | 15,4 | 20,5 |
| Huile de thon | 2 | 2 | 2 | 2 | 2 |
| Huile de soja | 1 | 0,85 | 0,75 | 0,65 | 0,55 |
| Lécithine de | 2 | 2 | 2 | 2 | 2 |
| Lysine | 0 | 0,1 | 0,2 | 0,3 | 0,4 |
| Méthionine | 0 | 0,05 | 0,1 | 0,15 | 0,2 |
| Phosphate monocalcique | 0 | 1,2 | 2,2 | 3,3 | 4,5 |
| Roche calcaire / coquille d'huître | 0 | 0 | 0 | 0,5 | 1 |
| Carbamide | 1,7 | 1,7 | 1,7 | 1,7 | 1,7 |
| Prémix de vitamines | 1,6 | 1,6 | 1,6 | 1,6 | 1,6 |
| **Somme** | 100,00 | 100,00 | 100,00 | 100,00 | 100,00 |
| Composition chimique par (AOAC, 2000) | | | | | |
| Humidité (%) | 9,38 | 8,82 | 8,75 | 9,02 | 9,45 |
| Protéines (%) | 35,30 | 35,66 | 35,73 | 36,39 | 36,37 |
| Lipides (%) | 7,18 | 7,16 | 7,18 | 7,06 | 7,06 |
| Fibre (%) | 2,86 | 2,86 | 2,85 | 2,79 | 2,78 |
| Cendre (%) | 8,25 | 8,02 | 7,35 | 6,65 | 6,03 |
| Énergie (Kcal/Kg) | 4470,38 | 4434,08 | 4404,79 | 4362,88 | 4318,97 |

Les données nécessaires pour cette expérience concernent la performance de croissances des crevettes dans des conditions de test. Celles-ci incluent :

✔ Performances de croissance.

- Le poids vif ou poids moyen de la crevette, le gain de poids
- Le taux de croissance spécifique (TCS : {(Ln poids Semaine 8) - (Ln poids Semaine 0)} X100 / jours de la période de culture)
- Le taux de conversion alimentaire (TCA : consommation d'aliments / production de crevettes)
- La survie (selon la méthode usuelle du laboratoire de recherche) / Taux de mortalité

✔ Statut immunitaire.

Le statut immunitaire est déterminé à la fin de l'étude par le comptage total des hémocytes, l'activité phénol-oxydase, les protéines de l'hémolymphe.

✔ Morphologie des villosités intestinales et de l'hépatopancréas.

A la fin de l'étude, une histopathologie des villosités intestinales et de l'hépatopancréas a été effectuée.

✔ Test de résistance face au pathogène *Vibrio parahaemolyticus.*

La résistance des crevettes blanches du Pacifique face au *Vibrio parahaemolyticus* a été étudiée afin de déterminer la capacité des crevettes à résister contre les bactéries pathogènes.

Après avoir alimenté les crevettes avec un régime alimentaire expérimental pendant 8 semaines, trente crevettes de chaque régime ont été échantillonnées et ont fait l'objet d'une injection de pathogènes, à savoir la bactérie *Vibrio parahaemolyticus,* 4,3.105 UFC (unité formant colonie)/ml (4,61ogUFC/ml) injectées par voie intramusculaire. La mortalité a été enregistrée durant 10 jours. La capacité de réduction bactérienne a été déterminée.

## 2. Résultats des Expériences 1, 2 et 3

### Résultats de l'Expérience 1

L'appétence de la poudre d'insectes dans le régime alimentaire de la crevette blanche *(Litopenaeus vannamei)* a été étudiée en se focalisant sur le temps de démarrage de la consommation des aliments par les crevettes, le nombre de crevettes consommant les aliments dans les 15 minutes et la quantité d'aliments consommée lors du repas observé. Les résultats de la capacité d'attraction de la poudre d'insectes ont été présentés dans le Tableau 6 ci-après. La capacité d'attraction, en termes de quantité d'aliments consommée lors du repas observé, le temps de démarrage de la consommation des aliments et le nombre de crevettes consommant les aliments, ne sont pas significativement différentes entre les différents régimes alimentaires (p> 0,05). La consommation alimentaire quotidienne par repas des crevettes nourries avec 30 % de farine de poisson (R1) semble être stable alors que les groupes de crevettes alimentées avec 15 % de farine de poisson avec et sans poudre d'insectes de *Tenebrio molitor* (R2-R5) présentent une forte variation de la consommation d'aliments par repas. Le temps nécessaire aux crevettes pour démarrer la consommation des aliments et le nombre de crevettes consommant les aliments sont tout à fait stables après cinq jours d'étude. Ces éléments peuvent signifier que le repas à base de poudre d'insectes de *Tenebrio molitor* n'a pas montré une meilleure attractivité dans les régimes alimentaires de la crevette blanche. De plus, cela met également en évidence que la diminution des farines de poisson dans les régimes alimentaires de la crevette blanche a un effet non significatif sur la consommation alimentaire des crevettes.

**Tableau 6** : Capacité d'attraction du repas à base de poudre d'insectes dans le régime alimentaire de la crevette blanche d'un taux d'inclusion de 15 % dans la farine de poisson

| Attraction | R1 INSM 0 % - FM 30 % | R2 INSM 0 % - FM 15 % | R3 INSM 5% - FM 10 % | R4 INSM 10 % - FM 5 % | R5 INSM 15 % - FM 0 % | Valeur-P |
|---|---|---|---|---|---|---|
| Consommation alimentaire (g/repas) | 1,32 | 1,25 | 1,18 | 1,12 | 1,19 | 0,340 |

(suite)

| Attraction | R1<br><br>INSM 0 % - FM 30 % | R2<br><br>INSM 0 % - FM 15 % | R3<br>INSM 5% - FM 10 % | R4<br><br>INSM 10 % - FM 5 % | R5<br><br>INSM 15 % - FM 0 % | Valeur-P |
|---|---|---|---|---|---|---|
| Temps de démarrage de la consommation alimentaire (seconde) | 31,35 | 30,18 | 27,35 | 31,13 | 28,78 | 0,685 |
| Nombre de crevettes consommant des aliments dans les 15 minutes | 5,40 | 5,28 | 4,85 | 4,38 | 4,80 | 0,307 |

## Résultats de l'Expérience 2

La digestibilité *in vivo* de la poudre d'insectes de *Tenebrio molitor* chez la crevette blanche a été déterminée par la méthode indirecte selon Cho et al. (1985). La digestibilité apparente des nutriments est présentée dans le Tableau 7 ci-après. Les résultats indiquent que la poudre d'insectes de *Tenebrio molitor*a une teneur élevée en protéines, lipides, et une digestibilité énergétique de 96 à 97 %.

**Tableau 7** : Digestibilité apparente des nutriments dans la poudre d'insectes selon l'Exemple 1 (moyenne $\pm$ ET)

| Digestibilité apparente | Poudre d'insectes |
|---|---|
| Digestibilité des protéines (%) | 97,47+1,66 |
| Digestibilité des lipides (%) | 97,85+0,36 |
| Digestibilité énergétiques (%) | 96,05+1,20 |

## Résultats de l'Expérience 3

• Efficacité de la poudre d'insectes sur la performance de la crevette blanche.

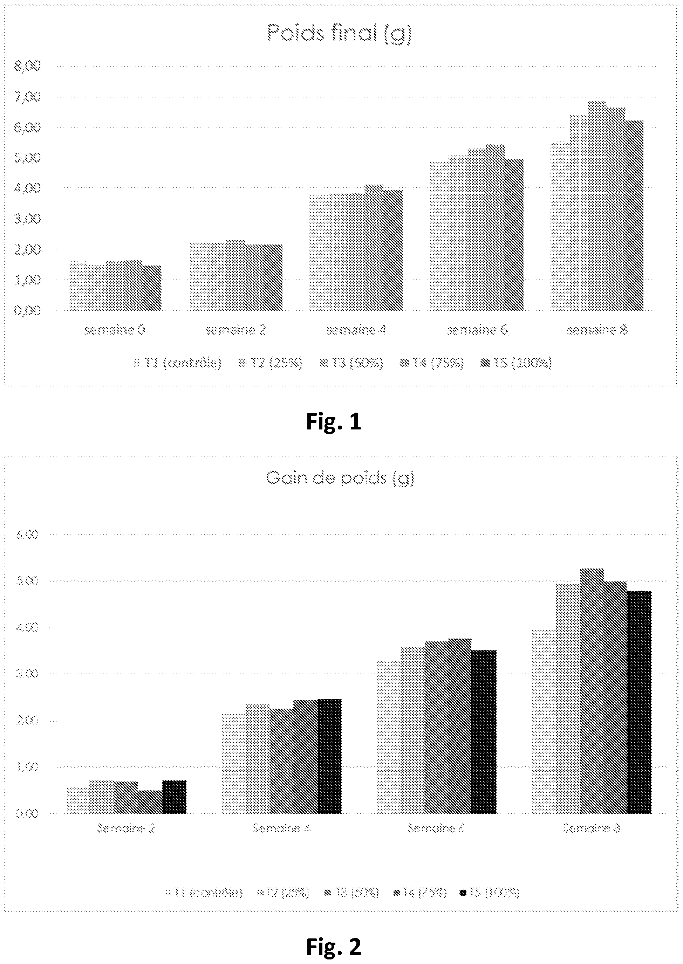

Les effets de la poudre d'insectes sur la performance de la crevette blanche *(L. vannamei)* ont été étudiés pendant 8 semaines. Les résultats sur la performance de croissance des crevettes ont été présentés dans le Tableau 8, ainsi qu'en Figures 1 et 2. Les résultats ont montré qu'à partir de la 8$^{ième}$ semaine, les performances de croissance des crevettes en termes de poids final, gain de poids, gain moyen quotidien et taux de croissance spécifique étaient significativement supérieures par rapport au contrôle (p <0,05). Les crevettes alimentées avec la poudre d'insectes avaient un meilleur taux de croissance que le contrôle (ledit contrôle présentant 25 % de farine de poisson, sans supplément de poudre d'insectes). La consommation alimentaire sur les 8 semaines n'étaient, en revanche, pas significativement différente (p> 0,05). Le taux de survie sur 8 semaines d'étude n'était également pas significativement différent (p> 0,05). Le taux de conversion alimentaire était significativement inférieur à partir de la semaine 8 pour T2-T5 par rapport au régime contrôle (p<0,05). Le ratio d'efficacité protéique n'est pas significativement différent (p> 0,05).
Par conséquent, la poudre d'insectes peut favoriser le taux de croissance de la crevette dans un régime alimentaire réduisant la farine de poisson jusqu'à un remplacement de 100 % (taux d'inclusion de farine de poisson 25 % dans le régime contrôle). La raison est la grande digestibilité des protéines et des lipides de poudre d'insectes.

**Tableau 8 :** Performances de croissance des crevettes blanches nourries avec un régime alimentaire comportant de la poudre d'insectes à la place de la farine de poisson et comparaison avec le contrôle (sans poudre d'insectes)

| Performances de croissance | Périodes | T1 INSM remplace 0 % de FM | T2 INSM remplace 25 % de FM de | T3 INSM remplace 50 % de FM | T4 INSM remplace 75 % de FM | T5 INSM remplace 100 % de FM | Valeur-P |
|---|---|---|---|---|---|---|---|
| Production (g/ aquarium) | 0 semaine | 24,03 [a] | 22,48 [a] | 24,00 [a] | 24,95 [a] | 21,87 [a] | 0,094 |
| | 2 semaines | 32,33 [a] | 32,48 [a] | 33,73 [a] | 32,18 [a] | 32,12[a] | 0,910 |
| | 4 semaines | 53,28 [a] | 55,10 [a] | 54,75 [a] | 59,62 [a] | 56,13 [a] | 0,493 |
| | 6 semaines | 62,75 [a] | 65,52 [a] | 68,82 [a] | 72,30 [a] | 63,20 [a] | 0,340 |
| | 8 semaines | 63,77 [b] | 72,22 [ab] | 79,00 [a] | 78,55 [a] | 71,62[ab] | 0,025 |
| Nombre de crevettes (par aquarium) | 0 semaine | 10 | 10 | 10 | 10 | 10 | / |
| Poids final (g / individu) | 0 semaine | 1,60 [a] | 1,50 [a] | 1,60 [a] | 1,66 [a] | 1,46 [a] | 0,098 |
| | 2 semaines | 2,21 [a] | 2,22 [a] | 2,29 [a] | 2,17 [a] | 2,17 [a] | 0,831 |
| | 4 semaines | 3,76 [a] | 3,86 [a] | 3,85 [a] | 4,11 [a] | 3,93 [a] | 0,662 |
| | 6 semaines | 4,88 [a] | 5,08 [a] | 5,29 [a] | 5,42 [a] | 4,97 [a] | 0,537 |
| | 8 semaines | 5,54 [b] | 6,43 [a] | 6,87 [a] | 6,65 [a] | 6,23 [ab] | 0,030 |
| Gain de poids (g / individu) | 2 semaines | 0,60 [a] | 0,72 [a] | 0,69 [a] | 0,51 [a] | 0,71 [a] | 0,213 |
| | 4 semaines | 2,16 [a] | 2,36 [a] | 2,25 [a] | 2,45 [a] | 2,47 [a] | 0,592 |
| | 6 semaines | 3,28 [a] | 3,58 [a] | 3,69 [a] | 3,76 [a] | 3,51 [a] | 0,665 |
| | 8 semaines | 3,94 [b] | 4,93 [a] | 5,27 [a] | 4,99 [a] | 4,77 [a] | 0,021 |
| Gain de poids quotidien (g/ individu/j) | 2 semaines | 0,04 [a] | 0,05 [a] | 0,05 [a] | 0,04 [a] | 0,05 [a] | 0,019 |
| | 4 semaines | 0,08 [a] | 0,08 [a] | 0,08 [a] | 0,09 [a] | 0,09 [a] | 0,628 |
| | 6 semaines | 0,08 [a] | 0,09 [a] | 0,09 [a] | 0,09 [a] | 0,08 [a] | 0,672 |
| | 8 semaines | 0,07 [b] | 0,09 [a] | 0,09 [a] | 0,09 [a] | 0,09 [a] | 0,007 |

(suite)

| Performances de croissance | Périodes | T1 INSM remplace 0 % de FM | T2 INSM remplace 25 % de FM de | T3 INSM remplace 50 % de FM | T4 INSM remplace 75 % de FM | T5 INSM remplace 100 % de FM | Valeur-P |
|---|---|---|---|---|---|---|---|
| Taux de croissance spécifique (%/j) | 2 semaines | 2,29 [a] | 2,81 [a] | 2,56 [a] | 1,88 [a] | 2,82 [a] | 0,087 |
| | 4 semaines | 3,05 [a] | 3,38 [a] | 3,12 [a] | 3,24 [a] | 3,52 [a] | 0,254 |
| | 6 semaines | 2,66 [a] | 2,89 [a] | 2,84 [a] | 2,81 [a] | 2,91 [a] | 0,577 |
| | 8 semaines | 2,22 [b] | 2,59 [a] | 2,60 [a] | 2,47 [a] | 2,59 [a] | 0,016 |
| Consommation alimentaire (g/individu) | 2 semaines | 0,53 [a] | 0,50 [a] | 0,53 [a] | 0,55 [a] | 0,48 [a] | 0,097 |
| | 4 semaines | 1,67 [a] | 1,63 [a] | 1,72 [a] | 1,65 [a] | 1,60 [a] | 0,695 |
| Performances de croissance | Périodes | T1 INSM remplace 0 % de FM | T2 INSM remplace 25 % de FM | T3 INSM remplace ^ 50 % de FM | T4 INSM remplace 75 % de FM | T5 INSM remplace 100 % de FM | Valeur-P |
| | 6 semaines | 4,04 [a] | 4,04 [a] | 4,07 [a] | 4,15 [a] | 4,14 [a] | 0,977 |
| | 8 semaines | 6,17 [a] | 6,40 [a] | 6,26 [a] | 6,46 [a] | 6,27 [a] | 0,975 |
| Consommation alimentaire quotidienne (g/individu/j) | 2 semaines | 0,04 [a] | 0,04 [a] | 0,04 [a] | 0,04 [a] | 0,03 [a] | 0,068 |
| | 4 semaines | 0,06 [a] | 0,06 [a] | 0,06 [a] | 0,06 [a] | 0,06 [a] | 0,605 |
| | 6 semaines | 0,10 [a] | 0,10 [a] | 0,10 [a] | 0,10 [a] | 0,10 [a] | 0,946 |
| | 8 semaines | 0,11 [a] | 0,11 [a] | 0,11 [a] | 0,12 [3] | 0,11 [a] | 0,985 |
| Taux de conversion alimentaire | 2 semaines | 0,90 [a] | 0,80 [a] | 0,84 [a] | 1,21 [a] | 0,69 [a] | 0,119 |
| | 4 semaines | 0,78 [a] | 0,71 [a] | 0,78 [a] | 0,68 [a] | 0,66 [a] | 0,270 |
| | 6 semaines | 1,24 [a] | 1,16 [a] | 1,11 [a] | 1,13 [a] | 1,20 [a] | 0,683 |
| | 8 semaines | 1,59 [a] | 1,32 [b] | 1,20 [b] | 1,30 [b] | 1,32 [b] | 0,052 |

(suite)

| Performances de croissance | Périodes | T1 INSM remplace 0 % de FM | T2 INSM remplace 25 % de FM | T3 INSM remplace ^ 50 % de FM | T4 INSM remplace 75 % de FM | T5 INSM remplace 100 % de FM | Valeur-P |
|---|---|---|---|---|---|---|---|
| Taux de survie (%) | 2 semaines | 97,78 [a] | 97,78 [a] | 97,78 [a] | 98,89 [a] | 98,89 [a] | 0,924 |
| | 4 semaines | 94,44 [a] | 95,56 [a] | 94,44 [a] | 96,67 [a] | 95,56 [a] | 0,909 |
| | 6 semaines | 85,56 [a] | 86,67 [a] | 86,67 [a] | 88,89 [a] | 84,44 [a] | 0,719 |
| | 8 semaines | 76,67 [a] | 75,56 [a] | 76,67 [a] | 78,89 [a] | 76,67 [a] | 0,896 |
| Ratio d'efficacité protéique | 10 semaines | 1,80 [a] | 2,13 [a] | 2,33 [a] | 2,12 [a] | 2,06 [a] | 0,106 |
| Remarque : Les moyennes avec les mêmes exposants au sein des lignes ne sont pas significativement différentes (p> 0,05). | | | | | | | |

• Immunité des crevettes.

L'immunité des crevettes dans des conditions normales a été étudiée sur 8 semaines d'expérience. Les résultats présentés dans le Tableau 9 ont montré qu'il n'y avait pas de différence significative par rapport au contrôle (p> 0,05) sur le nombre total des hémocytes, la teneur protéique de l'hémolymphe et l'activité phénol-oxydase. Par conséquent, la poudre d'insectes peut remplacer la farine de poisson jusqu'à 100 % (taux d'inclusion de 25 % de farine de poisson dans la formule alimentaire) sans aucun effet indésirable sur l'immunité des crevettes

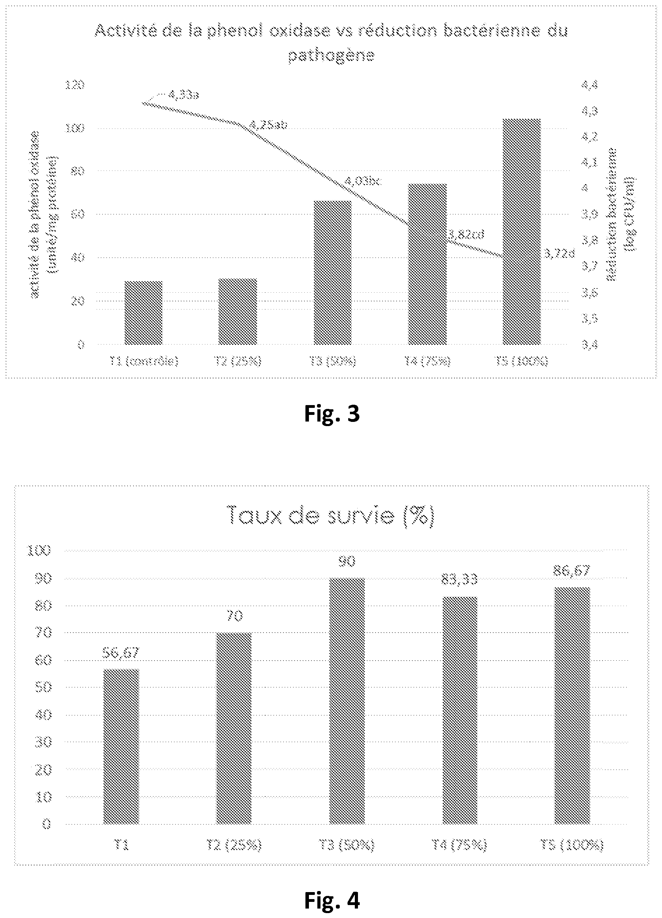

La condition de résistance après l'injection de *Vibrio parahemolyticus* est étudiée pendant 6 h. L'immunité de la crevette dans le Tableau 9 montre une grande (p <0,05) activité du phénol-oxydase dans le groupe des crevettes nourries avec la poudre d'insectes remplaçant 50 à 100 % de la farine de poisson (taux d'inclusion de la farine de poisson dans le régime alimentaire de 10,3 à 20,6 %). Plus particulièrement, sur la Figure 3, on constate une corrélation négative entre la présence bactérienne et l'activité de la phénol oxydase. De plus, il y a une corrélation forte et négative entre la diminution bactérienne et l'augmentation de l'inclusion de la poudre d'insecte dans le régime alimentaire (p<0,05).

**Tableau 9** : Immunité des crevettes blanches nourries de poudre d'insectes de *Tenebrio molitor* en remplacement de la farine de poisson et comparaison avec le contrôle (sans poudre d'insectes)

| Immunité | Condition | T1 INSM remplace 0 % de FM | T2 INSM remplace 25 % de FM | T3 INSM remplace 50 % de FM | T4 INSM remplace 75 % de FM | T5 INSM remplace 100 % de FM | Valeur-P |
|---|---|---|---|---|---|---|---|
| Nombre total des hémocytes (x10$^6$ cellules / ml) | Normal | 1,01[a] | 1,24 [a] | 1,16 [a] | 1,28 [a] | 1,31 [a] | 0,160 |
| | Injection | 0,89 [a] | 1,16 [a] | 1,03 [a] | 1,16 [a] | 1,10 [a] | 0,299 |
| Teneur protéique de l'hémolymphe (g/dl) | Normal | 4,54 [a] | 5,71 [a] | 4,93 [a] | 6,24 [a] | 6,01 [a] | 0,674 |
| | Injection | 5,83 [a] | 6,21 [a] | 4,99 [a] | 3,53 [a] | 4,06 [a] | 0,066 |
| Activité phénol-oxydase (unité/mg protéine) | Normal | 113,24 [a] | 141,70 [a] | 135,43 [a] | 137,93 [a] | 121,98 [a] | 0,961 |
| | Injection | 29,29[c] | 30,24[c] | 66,29[b] | 74,32[ab] | 104,64[a] | 0,002 |

(suite)

| Immunité | Condition | T1 INSM remplace 0 % de FM | T2 INSM remplace 25 % de FM | T3 INSM remplace 50 % de FM | T4 INSM remplace 75 % de FM | T5 INSM remplace 100 % de FM | Valeur-P |
|---|---|---|---|---|---|---|---|
| Capacité de réduction bactérienne (ou clairance) (Log UFC / ml) | Injection | 4,33 a | 4,25 ab | 4,03 bc | 3,82 cd | 3,72 d | 0,001 |
| Remarque : Les moyennes avec les mêmes exposants au sein des lignes ne sont pas significativement différentes (p> 0,05). | | | | | | | |

• La mortalité des crevettes

La mortalité des crevettes après une exposition à *Vibrio parahaemolyticus,* $4,3 \times 10^5$ ufc/ml, a été présentée dans le Tableau 10, ainsi qu'en Figure 4. Les crevettes nourries avec des régimes alimentaires dont la farine de poisson a été réduite (de 50 à 100 %) et supplémentée par de la poudre d'insectes de *Tenebrio molitor* (remplaçant la farine de poisson de 50 à 100 %) présentaient un taux de mortalité significativement plus faible (P <0,05) que le régime alimentaire de contrôle avec 25 % de farine de poisson (T1) et que le groupe de crevettes nourri avec des régimes alimentaires dont la farine de poisson a été réduite et supplémentée par la poudre d'insectes (25 % de farine de poisson remplacée). Ceci peut provenir de la haute digestibilité lipidique et protéique de la poudre d'insectes et d'une meilleure qualité possible des lipides par rapport à l'huile de soja permettant aux crevettes d'obtenir plus de nutriments.

**Tableau 10** : Taux de mortalité cumulé après l'injection du pathogène *Vibrio parahaemolyticus* chez les crevettes blanches nourries avec des régimes alimentaires comportant de la poudre d'insectes à la place de la farine de poisson et comparaison avec le contrôle (sans poudre d'insectes)

| Jour(s) après le l'injection du pathogène | T1 INSM remplace 0 % de FM | T2 INSM remplace 25 % de FM | T3 INSM remplace 50 % de FP | T4 INSM remplace 75 % de FM | T5 INSM remplace 100 % de FM | Valeur-P |
|---|---|---|---|---|---|---|
| Jour 1 | 0,00 | 0,00 | 0,00 | 0,00 ab | 0,00 | * |
| Jour 2 | 10,00 a | 3,33 ab | 0,00 b | 6,67 ab | 0,00 b | 0,029 |
| Jour 3 | 23,33 a | 13,33 ab | 3,33 b | 13,33 ab | 3,33 b | 0,008 |
| Jour 4 | 30,00 a | 23,33 ab | 6,67 b | 13,33 ab | 10,00 b | 0,048 |
| Jour 5 | 40,00 a | 30,00 ab | 10,00 b | 13,33 ab | 13,33 ab | 0,014 |
| Jour 6 | 40,00 a | 30,00 ab | 10,00 b | 13,33 ab | 13,33 ab | 0,014 |
| Jour 7 | 40,00 a | 30,00 ab | 10,00 C | 16,67 bc | 13,33 bc | 0,017 |
| Jour 8 | 40,00 a | 30,00 ab | 10,00 c | 16,67 bc | 13,33 bc | 0,017 |
| Jour 9 | 40,00 a | 30,00 ab | 10,00 c | 16,67 bc | 13,33 bc | 0,017 |
| Jour 10 | 43,33 a | 30,00 ab | 10,00 c | 16,67 bc | 13,33 bc | 0,005 |
| Taux de mortalité (%) | 43,33 a | 30,00 ab | 10,00 c | 16,67 bc | 13,33 bc | 0,005 |
| Taux de survie (%) | 56,67 c | 70,00 bc | 90,00 a | 83,33 ab | 86,67 ab | 0,005 |
| Remarque : Les moyennes avec les mêmes exposants au sein des lignes ne sont pas significativement différentes (p> 0,05). * aucune analyse statistique, la valeur est la même. | | | | | | |

• Histologie de l'hépatopancréas et des villosités intestinales des crevettes.

L'histologie hépato-pancréatique des crevettes nourries avec le régime alimentaire de contrôle et les régimes avec la poudre d'insectes ont montré des cellules B et cellules R plus grandes. Ces cellules produisent des enzymes de régulation des nutriments. Dans les régimes alimentaires T2-T5, les cellules B (sécrétrices) et R (de réserve) grandissent plus que dans le régime de contrôle T1. Cela signifie que les cellules hépatocytaires ayant accumulé davantage de nutriments peuvent être des réserves de glycogène et/ou lipidiques et ceci en raison de la haute digestibilité protéique ou lipidique de la poudre d'insectes. La densité et la longueur de villosités intestinales étaient élevées dans tous les régimes alimentaires.

### 3. Conclusion

La poudre d'insectes, en particulier de *Tenebrio molitor,* peut remplacer la farine de poisson et ce jusqu'à 100 % de remplacement de la farine de poisson, sans aucun effet négatif sur les performances de croissance, la santé des crevettes et l'histologie de l'hépatopancréas et des villosités intestinales chez la crevette de petite taille (1-5 g) avec un profil nutritionnel équivalent entre les régimes alimentaires comprenant de la farine de poisson et/ou de la poudre d'insectes. Lorsque la poudre d'insectes remplace entre 25 et 100 % de la farine de poisson dans le régime alimentaire chez la crevette durant plus de 6 semaines, la crevette peut présenter le meilleur taux de croissance et une digestibilité élevée. Le groupe des crevettes nourries de poudre d'insectes de *Tenebrio molitor* en remplacement de la farine de poisson à 50 ou 100 %, a montré une meilleure résistance contre le pathogène *Vibrio parahaemolyticus.*

### Revendications

1.  Poudre de coléoptères pour une utilisation dans le traitement de la vibriose chez les bivalves et les décapodes, les coléoptères appartenant aux familles des Tenebrionidae, Melolonthidae, Dermestidae, Coccinellidae, Cerambycidae, Carabidae, Buprestidae, Cetoniidae, Dryophthoridae, ou leurs mélanges.

2.  Poudre de coléoptères pour une utilisation selon la revendication 1, dans le traitement de l'entérite de la crevette.

3.  Poudre de coléoptères pour une utilisation selon la revendication 2, dans le traitement du syndrome de mortalité précoce de la crevette / syndrome de nécrose hépatopancréatique aiguë chez la crevette.

4.  Poudre de coléoptères pour une utilisation selon l'une quelconque des revendications 1 à 3, comportant au moins 67% en poids de protéines et au moins 0,1% en poids de chitine, les pourcentages étant donnés sur le poids total de poudre de coléoptères.

5.  Poudre de coléoptères pour une utilisation selon la revendication 4, comportant au moins 67% en poids de protéines et au moins 5% en poids de chitine, les pourcentages étant donnés sur le poids total de poudre de coléoptères.

6.  Poudre de coléoptères pour une utilisation selon la revendication 4, comportant au moins 71% en poids de protéines et comprenant entre 0,1 et 2% en poids de chitine, les pourcentages étant donnés sur le poids total de poudre de coléoptères.

7.  Poudre de coléoptères pour une utilisation selon l'une quelconque des revendications 1 à 6, obtenue à partir de l'espèce *Tenebrio Molitor.*

### Patentansprüche

1.  Coleopterapulver zur Verwendung bei der Behandlung von Vibriose bei Bivalvia und Decapoda, wobei die Coleoptera zu den Familien der Tenebrionidae, Melolonthidae, Dermestidae, Coccinellidae, Cerambycidae, Carabidae, Buprestidae, Cetoniidae, Dryophthoridae, oder Mischungen davon, gehören.

2.  Coleopterapulver zur Verwendung nach Anspruch 1 bei der Behandlung von Enteritis bei Garnelen.

3.  Coleopterapulver zur Verwendung nach Anspruch 2 bei der Behandlung des Frühsterblichkeitssyndroms bei Garnelen / akuten hepatopankreatischen Nekrose-Syndroms bei Garnelen.

**4.** Coleopterapulver zur Verwendung nach einem der Ansprüche 1 bis 3, das mindestens 67 Gewichts-% Proteine und mindestens 0,1 Gewichts-% Chitin umfasst, wobei sich die Prozentangaben auf das Gesamtgewicht des Coleopterapulvers beziehen.

**5.** Coleopterapulver zur Verwendung nach Anspruch 4, das mindestens 67 Gewichts-% Proteine und mindestens 5 Gewichts-% Chitin umfasst, wobei sich die Prozentangaben auf das Gesamtgewicht des Coleopterapulvers beziehen.

**6.** Coleopterapulver zur Verwendung nach Anspruch 4, das mindestens 71 Gewichts-% Proteine umfasst und zwischen 0,1 und 2 Gewichts-% Chitin umfasst, wobei sich die Prozentangaben auf das Gesamtgewicht des Coleopterapulvers beziehen.

**7.** Coleopterapulver zur Verwendung nach einem der Ansprüche 1 bis 6, das aus der Art *Tenebrio Molitor* gewonnen wird.

**Claims**

**1.** Beetle powder for use in the treatment of vibriosis in bivalves and decapods, the beetles belong to the families of the Tenebrionidae, Melolonthidae, Dermestidae, Coccinellidae, Cerambycidae, Carabidae, Buprestidae, Cetoniidae, Dryophthoridae, or mixtures thereof.

**2.** Beetle powder for use according to claim 1, in the treatment of shrimp enteritis.

**3.** Beetle powder for use according to claim 2, in the treatment of shrimp early mortality syndrome/acute hepatopancreatic necrosis syndrome in shrimps.

**4.** Beetle powder for use according to any one of claims 1 to 3, comprising at least 67% by weight proteins and at least 0.1% by weight chitin, percentages being given with respect to the total weight of beetle powder.

**5.** Beetle powder for use according to claim 4, comprising at least 67% by weight proteins and at least 5% by weight chitin, percentages being given with respect to the total weight of beetle powder.

**6.** Beetle powder for use according to claim 4, comprising at least 71% by weight proteins and comprising between 0.1 and 2% by weight chitin, percentages being given with respect to the total weight of beetle powder.

**7.** Beetle powder for use according to any one of claims 1 to 6, obtained from the species *Tenebrio molitor.*

**Fig. 1**

**Fig. 2**

**Fig. 3**

**Fig. 4**